# FASCICULE DE BREVET EUROPEEN

(11) **EP 4 392 399 B1**
(45) Date de publication et mention de la délivrance du brevet: **14.01.2026**
(21) Numéro de dépôt: 22757315.1
(22) Date de dépôt: 25.07.2022
(51) Int. Cl.: C07C 67/08, C07C 67/54, C07C 69/54, C07C 67/60, C07C 67/327

(54) **PROCEDE PERFECTIONNE DE FABRICATION D'ACRYLATE DE BUTYLE DE PURETE ELEVEE**
VERBESSERTES VERFAHREN ZUR HERSTELLUNG VON HOCHREINEM BUTYLACRYLAT
IMPROVED METHOD FOR PRODUCING HIGH-PURITY BUTYL ACRYLATE

(30) Priorité: 25.08.2021 FR 2108885
(43) Date de publication de la demande: 03.07.2024
(73) Titulaire: ARKEMA FRANCE, 92800 Puteaux (FR)
(72) Inventeur: TRETJAK, Serge, 57501 SAINT AVOLD cedex (FR); LEVRAY, André, 57501 SAINT AVOLD cedex (FR)
(74) Mandataire: Arkema Patent
(86) Numéro de dépôt international: PCT/FR2022/051488
(87) Numéro de publication internationale: WO 2023/025999

(56) Documents cités:
- FR-A1- 3 032 198
- JP-A- 2015 140 336

## Description

### DOMAINE TECHNIQUE

La présente invention concerne la fabrication d'acrylate de butyle par estérification directe de l'acide acrylique par du butanol, cette réaction étant catalysée par de l'acide sulfurique. Plus spécifiquement, elle a pour objet un procédé perfectionné de fabrication d'acrylate de butyle, comprenant une étape de valorisation des sous-produits lourds générés au cours de cette fabrication, conduisant à une productivité élevée d'un produit répondant aux normes en matière de pureté et d'acidité, dans des conditions énergétiques optimisées.

### ARRIERE-PLAN TECHNIQUE ET PROBLEME TECHNIQUE

L'estérification de l'acide acrylique est une réaction équilibrée avec génération d'eau qu'il est nécessaire d'éliminer au cours de la réaction pour déplacer l'équilibre dans le sens de la production de l'ester acrylique.

Les problèmes qui se posent lors de la fabrication de l'acrylate de butyle par estérification directe de l'acide acrylique, généralement en présence d'acide sulfurique comme catalyseur, sont le plus souvent liés à la complexité des étapes de purification nécessaires après l'étape réactionnelle pour obtenir un produit de haute pureté, au détriment de la productivité du procédé.

Le procédé industriel, tel que décrit dans le brevet EP 609127 de la demanderesse, consiste à estérifier l'acide acrylique par du butanol en excès, en présence d'acide sulfurique. Le mélange réactionnel en fin de réaction comprend de l' acrylate de butyle, de l'acide acrylique résiduel, du sulfate acide de butyle, des traces d'acide sulfurique et différentes impuretés résultant de réactions secondaires. Ce mélange réactionnel est ensuite soumis à une étape de neutralisation et de lavage à l'eau qui a pour objet d'éliminer les impuretés dites acides : acide sulfurique résiduel, sulfate acide de butanol et acide acrylique. Ce mélange exempt d'impuretés acides est soumis à différentes étapes de purification, qui conduisent à la récupération de l'acrylate de butyle purifié. Une des étapes dite d'étêtage consiste notamment à distiller le butanol et les sous- produits légers. Le butanol peut être ainsi recyclé à la réaction d'estérification.

L'étape finale de purification de l'acrylate de butyle consiste à envoyer le mélange contenant de l'ester débarrassé des produits légers dans une dernière colonne de distillation d'où il sort en tête, purifié des sous-produits lourds qui eux se retrouvent en pied de la colonne de distillation et sont ensuite concentrés dans un évaporateur.

Parmi les sous-produits générés selon les réactions secondaires, on peut citer les produits légers tels que l'acétate de butyle, le propionate de butyle, l'éther dibutylique, l' acrylate d'isobutyle ou les produits lourds comme le maléate de dibutyle.

Les composés « lourds » issus de réactions d'addition de Michael se forment spontanément dans les unités de production d'acrylate de butyle Ces réactions parasites sont favorisées par des températures élevées rencontrées en particulier dans les pieds de colonnes à distiller de ces unités. Ainsi l'acide acrylique, le butanol n'ayant pas encore réagi ou l'eau de réaction s'ajoutent à la double liaison de l'acrylate de butyle pour former principalement :
- l'acryloxypropionate de butyle (AA/ABU) par addition de l'acide acrylique (AA) sur l'acrylate de butyle (ABU) ;
- l'hydroxypropionate de butyle (HPB) par addition d'eau sur l'acrylate de butyle ;
- le butoxypropionate de butyle (BPB) par addition de butanol sur l'acrylate de butyle.

Une polyaddition ou la formation de composés mixtes est également possible.

Une des caractéristiques des sous-produits lourds est que leur point d'ébullition se trouve au-dessus des points d'ébullition de l'acide acrylique, du butanol et de l' acrylate de butyle. Comme leur volatilité est faible, ils s'accumulent en fond de la dernière colonne de distillation, en pied de l'évaporateur servant à concentrer ce résidu.

Le résidu de l'évaporateur, outre les dérivés de Michael et quelques pourcents de monomères libres, contient également une forte concentration en inhibiteurs de polymérisation, accumulés au fur et à mesure des étapes de purification, comme la phénothiazine sous sa forme libre ou d'adduit de l'AA ou de l'ABU, ainsi que des composés lourds de nature polymérique plus ou moins solubles dans le milieu. En général ce résidu est éliminé par incinération, ce qui entraîne une perte de rendement importante.

Différentes solutions ont été proposées pour la valorisation de ces sous-produits lourds.

Le document CN1063678 propose des méthodes de traitement des oxy-esters formés lors de la synthèse d'acrylate de butyle en utilisant des catalyseurs acides protiques comme l'acide sulfurique ou l'acide para toluène sulfonique. Des composés tels des phtalates peuvent également être ajoutés, comme décrit dans le document US 4293347.

Le désavantage de ces méthodes de craquage est que le produit résiduel est visqueux et contient des solides. US 6617470 propose d'utiliser comme catalyseurs, des acides arylsulfoniques comme l'acide dodécylsulfonique, ce qui supprime la formation de solides dans le résidu de pied.

Le document US 2011/0230675 propose d'ajouter de l'eau en continu lorsque le craquage est effectué par catalyse acide, afin d'éviter la formation de dépôt solide.

Le document FR 2901272 propose de réaliser une distillation avant craquage, ce qui limite les dépôts de matières solides dans les installations.

La société demanderesse a décrit dans son brevet FR 2727964 ou dans sa demande PCT/FR2021/050825 un craquage thermique d'un mélange de sous-produits d'acide acrylique (AA) et d'esters acryliques en vue de leur recyclage dans l'atelier de production de l'ester acrylique. Elle utilise la propriété des dérivés alcoxy-propioniques comportant des liaisons C-O-C, tel que le 3-ethoxypropionate d'éthyle, d'être plus difficiles à casser que les liaisons C-C des sous-produits lourds dérivés de l'acide acrylique.

La société demanderesse a décrit dans sa demande FR2101402 un schéma de procédé combinant une colonne à soutirage latéral, un craqueur thermique ou thermique et catalytique et un système de décanteur et de lavage pour traiter les produits issus du craqueur pour la synthèse d'ester acrylique tel l'acrylate de 2-éthylhexyle. Cette combinaison est rendue nécessaire car, dans ces procédés catalysés par des résines acides, les impuretés dites acides ne sont d'une part pas éliminées lors d'une opération de neutralisation préalablement à la section de purification et, d'autre part, sont également générées lors du craquage. Sans la combinaison : colonne à soutirage latéral - décanteur (voir exemple 2), il n'est pas possible d'obtenir un acrylate de 2-éthylhexyle purifié conforme aux spécifications.

Cependant, la transposition du schéma de procédé tel qu'envisagé dans le document FR2101402, pour la synthèse d'acrylate de butyle, entraînerait des pertes des matières valorisables issues du craquage, comme l'acide acrylique, et surtout du butanol qui sera en partie solubilisé dans de l'eau, lors du lavage du produit de tête craqueur dans le décanteur.

Le document JP 2015/140336 décrit un procédé de fabrication d'acrylate de butyle par estérification directe de l'acide acrylique par du butanol en présence d'acide comme catalyseur, conduisant à l'obtention d'un mélange réactionnel brut contenant de l'acrylate de butyle, de l'acide acrylique, du butanol résiduel et des impuretés.

Le document FR 3032198 décrit un procédé de préparation de (méth)acrylate d'alkyle par transestérification suivie d'étapes de purification par distillation.

Enfin le document CN102173990 propose d'ajouter dans l'alimentation du craqueur des sels de cuivre afin de faciliter le traitement ultérieur du résidu ultime de craquage.

Il subsiste donc un besoin d'améliorer les procédés de fabrication d'acrylate de butyle connus, afin d'obtenir le plus possible de produits valorisables : acide acrylique, butanol et acrylate de butyle, tout en évitant les problèmes de formation de solides. Par ailleurs, il est souhaitable de limiter la formation de sous-produits qui pourraient être difficiles à séparer dans les étapes de purification conduisant à l'obtention de l'acrylate de butyle purifié. Ces étapes de purification sont effectuées par distillation. Le tableau 1 comparant les températures d'ébullition à pression atmosphérique des sous-produits légers formés à la réaction permet de repérer les impuretés dont la formation sera impérativement à limiter lors de ce craquage.

**[Tableau 1]**

| | Température d'ébullition (°C) |
|---|---|
| *Sous-produits formés* | |
| Acétate de butyle | 126 |
| Ether dibutylique | 141 |
| Propionate de butyle | 146 |
| Acrylate d'isobutyle | 132 |
| | |

| *Réactifs et Produits* | |
|---|---|
| Acrylate de Butyle | 148 |
| Acide Acrylique | 141 |
| Butanol | 118 |

Il a maintenant été découvert que la mise en œuvre d'un craquage thermique sans catalyseur permet de transformer avec un haut rendement des adduits de Michael, sans dépôts solides dans l'installation et en limitant en particulier la formation d'éther dibutylique, dans un procédé permettant l'obtention d'acrylate de butyle de haute pureté.

### RESUME DE L'INVENTION

La présente invention décrit un traitement thermique permettant de valoriser les adduits de Michael dans un procédé permettant l'obtention d'acrylate de butyle comme décrit dans le brevet EP 609127 pour la partie réactionnelle.

Elle complète également le schéma de purification décrit dans ce brevet en associant un réacteur permettant de réaliser le craquage thermique des produits de purge du pied de l'évaporateur placé au bas de la colonne de purification de l'acrylate de butyle et indique le recyclage des produits de tête issus du craquage dans le procédé.

Elle est basée sur deux hypothèses :
- c'est l'interaction entre la phénothiazine et le catalyseur acide qui est grande partie responsable de la formation de coke lors de cette réaction de craquage, et
- la quantité d'éther dibutylique augmente lorsque le craquage est effectué en présence de catalyseur, ce qui accentue la réaction de déshydratation du butanol.

L'invention a pour objet un procédé de fabrication d'acrylate de butyle par estérification directe de l'acide acrylique par du butanol en excès en présence d'acide sulfurique comme catalyseur, conduisant à l'obtention d'un mélange réactionnel brut contenant de l'acrylate de butyle, acide acrylique et butanol résiduels, sulfate acide de butyle, des traces d'acide sulfurique et des impuretés résultant des réactions secondaires, ledit procédé comprenant des étapes de neutralisation et de lavage à l'eau conduisant à l'obtention d'un mélange réactionnel exempt d'impuretés dites acides, caractérisé en ce que ledit mélange réactionnel lavé d'impuretés acides est soumis au moins aux étapes i), ii) et iii) suivantes :
i) étêtage dans une colonne de distillation permettant d'obtenir :
   - en tête un flux composé essentiellement des réactifs non réagis ;
   - en pied un flux comprenant l'ester recherché et des sous-produits lourds ;
ii) on soumet le flux de pied de la colonne d'étêtage à une colonne de rectification permettant de séparer :
   - en tête l'ester recherché purifié ;
   - en pied un flux contenant des sous-produits lourds, qui est concentré sur un évaporateur à film ou distillé dans une colonne d'équeutage afin de recycler à la colonne de rectification les composés légers présents, et d'éliminer un résidu final de sous-produits lourds,
iii) on soumet le flux de pied de la colonne de rectification à un traitement thermique effectué en l'absence de catalyseur dans un craqueur placé en sortie de l'évaporateur, permettant de séparer :
   - en tête, un flux de produits valorisables recyclé à l'alimentation de la colonne d'étêtage;
   - en pied, un résidu envoyé vers une station de traitement.

La présente invention permet de surmonter les inconvénients de l'état de l'art. Elle fournit plus particulièrement un procédé permettant d'obtenir un acrylate de butyle de haute pureté ayant comme spécifications une pureté en ester supérieure à 99,5%, une teneur en éther dibutylique inférieure à 500 ppm, et enfin une teneur en eau inférieure à 400 ppm, intégrant un procédé thermique permettant d'effectuer le craquage des adduits de Michael en réactifs (acide acrylique et alcool) et en produit fini, augmentant ainsi la productivité du procédé en limitant la quantité de résidu à éliminer.

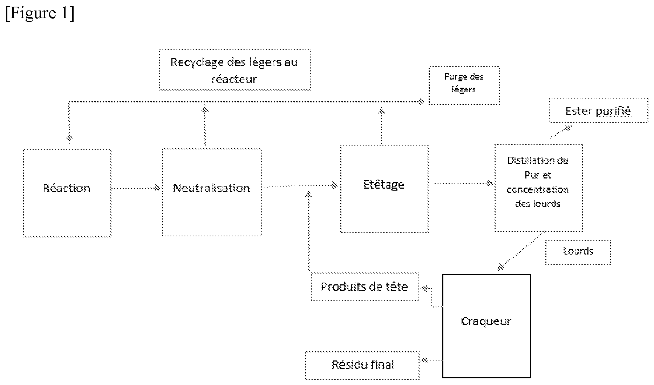

D'autres caractéristiques et avantages de l'invention ressortiront mieux à la lecture de la description détaillée qui suit, en référence à la Figure 1 annexée.
[Fig. 1] : schéma global du procédé de synthèse de l'acrylate de butyle selon l'invention, intégrant une étape de craquage thermique.

### DESCRIPTION DETAILLEE DE L'INVENTION

L'invention a pour objet un procédé de fabrication d'acrylate de butyle par estérification directe de l'acide acrylique par du butanol en excès, en présence d'acide sulfurique comme catalyseur, conduisant à l'obtention d'un mélange réactionnel brut contenant de l'acrylate de butyle, acide acrylique et butanol résiduels, sulfate acide de butyle, des traces d'acide sulfurique et des impuretés résultant des réactions secondaires.

Selon diverses réalisations, ledit procédé comprend les caractères suivants, le cas échéant combinés.

Après l'étape d'estérification, le procédé selon l'invention comprend des étapes de neutralisation et de lavage à l'eau conduisant à l'obtention d'un mélange réactionnel débarrassé des impuretés dites acides.

Par impuretés acides on comprend généralement l'acide sulfurique, le sulfate acide de butyle, le dimère d'acide acrylique et acide acrylique résiduel.

Selon un mode de réalisation, l'étape d'estérification est suivie par l'addition audit mélange réactionnel brut d'une base pour neutraliser l'acide acrylique, le sulfate acide de butyle, les traces d'acide sulfurique qui y sont présents, les sels résultants passant dans la phase aqueuse dudit mélange, la phase organique et la phase aqueuse issues de cette neutralisation étant séparées et l'acrylate de butyle recherché étant récupéré à partir de ladite phase organique.

On conduit l'étape de récupération de l'acrylate de butyle également de façon classique en lavant à l'eau, dans une colonne d'extraction, la phase organique issue de la séparation de phases qui suit la première neutralisation,

De manière caractéristique, le mélange réactionnel lavé d'impuretés acides comme décrit cidessus est soumis au moins aux étapes i), ii) et iii) suivantes :
i) étêtage dans une colonne de distillation permettant d'obtenir :
   - en tête un flux composé essentiellement des réactifs non réagis ;
   - en pied un flux comprenant l'ester recherché et des sous-produits lourds ;
ii) on soumet le flux de pied de la colonne d'étêtage à une colonne de rectification permettant de séparer :
   - en tête l'ester recherché purifié ;
   - en pied un flux contenant des sous-produits lourds, qui est concentré sur un évaporateur à film ou distillé dans une colonne d'équeutage afin de recycler à la colonne de rectification les composés légers présents, et d'éliminer un résidu final de sous-produits lourds,
iii) on soumet le flux de pied de la colonne de rectification à un traitement thermique effectué en l'absence de catalyseur dans un craqueur placé en sortie de l'évaporateur, permettant de séparer :
   - en tête, un flux de produits valorisables recyclé séparément à l'alimentation de la colonne d'étêtage ;
   - en pied, un résidu envoyé vers une station de traitement.

Selon un mode de réalisation, un craqueur thermique est placé en pied d'une colonne de purification permettant d'obtenir en tête de celle-ci l' acrylate de butyle et en pied les lourds que l'on va d'abord concentrer sur un évaporateur. Ce flux concentré sert à alimenter le craqueur thermique. Optionnellement, sans que cela nuise au fonctionnement du craqueur, des traitements préalables du flux d'alimentation, comme une distillation telle que décrite dans le brevet FR2901272, peuvent être mise en œuvre. Le flux de produits valorisables est recyclé à l'alimentation de la section permettant le recyclage de l'alcool à la réaction. Le résidu de pied de craqueur est envoyé vers une station de traitement.

Dans cette invention, la décomposition des adduits de Michael peut être réalisée suivant un mode continu, semi-continu ou batch. Le mode continu est préférable car il correspond au fonctionnement préféré de ce procédé d'estérification. On peut utiliser un réacteur tubulaire, un réacteur agité avec une double enveloppe ou ayant une boucle de chauffage externe avec une circulation forcée. Les composés valorisables générés par la réaction de craquage sont recueillis après condensation des vapeurs en tête du réacteur ou en tête d'une colonne à distiller surmontant ce dernier.

Température de réaction et pression au-dessus du réacteur sont reliées de façon à ce que l'on élimine par évaporation les réactifs tels l'acide acrylique, le butanol ou le produit final tout en maintenant dans le milieu réactionnel le butoxypropionate de butyle (BPB) qui est le principal composé (> 70% en poids) présent dans l'alimentation du craqueur.

Selon un mode de réalisation, la réaction de décomposition est effectuée dans une gamme de température de 220°C à 300°C et plus spécialement entre 230°C to 280°C.

Selon un mode de réalisation, la pression maintenue au-dessus du réacteur est comprise entre 50000 Pa à 300000 Pa.

Selon un mode de réalisation, la composition massique du produit d'alimentation du craqueur dans le cas de la fabrication de l'acrylate de butyle en présence de phénothiazine comme inhibiteur de polymérisation est la suivante :
- Butanol < 0,1%
- Acrylate de butyle (5-10%)
- Hydroxypropionate de butyle (HPB) : 1-3%
- Butoxypropionate de butyle (BPB) 70-80%
- Acryloxypropionate de butyle (AA/ABU) 4-6%
- Dibutylmaléate : 2-5%
- Phénothiazine : 1-3%.

Le traitement thermique s'effectue en l'absence de catalyseur.

Le temps de séjour dans le craqueur basé sur le débit d'alimentation (kg/h) rapporté au volume de la phase liquide dans le réacteur est choisi de préférence entre 0,5 to 20 heures, et spécialement entre 7 et 15 heures.

En référence à la Figure 1, qui représente le mode d'invention préféré, la section d'étêtage comporte une colonne à distiller possédant un équivalent de 10 et 30 plateaux théoriques de préférence 10 à 15 étages théoriques. Les internes utilisés pour la colonne peuvent être des plateaux à clapets ou des plateaux perforés à déversoir, des plateaux à courants croisés comme les Dual Flow les Ripple Trays, les Turbo Grid Shell, ou du garnissage ordonné comme du garnissage structuré tel le Mellapack 250X de Sulzer.

L'alimentation de la colonne d'étêtage est réalisée au tiers supérieur de cette colonne de préférence entre les plateaux théoriques 3 à 10 comptées à partir de la tête de la colonne. Le flux de tête de la colonne comprend essentiellement les réactifs non réagis. Ce flux valorisable est recyclé à la réaction.

La colonne fonctionne avec un taux de reflux (débit de liquide condensé retourné à la colonne/ débit recyclé à la réaction) compris entre 4/1 à 1/1 de préférence 3/1. Avantageusement, on introduit de 50 à 5000 ppm d'inhibiteur de polymérisation dans le système de purification selon le procédé de l'invention.

Comme inhibiteurs de polymérisation utilisables, on peut citer par exemple la phénothiazine (PTZ), l'hydroquinone (HQ), l'éther mono méthylique d'hydroquinone (EMHQ), le di-tert-butyl para-crésol (BHT), la paraphénylène diamine, le TEMPO (2,2,6,6-tétraméthyl-1-piperidinyloxy), le di-tert-butylcatéchol, ou les dérivés du TEMPO, tel que le OH-TEMPO, seuls ou leurs mélanges en toutes proportions, à des teneurs dans le milieu de réaction pouvant être comprises entre 50 ppm et 5000 ppm, éventuellement en présence d'air appauvri, mais généralement à des teneurs comprises entre 150 ppm et 1000 ppm. L'ajout des inhibiteurs de polymérisation peut se faire à différents endroits, avec l'introduction des réactifs ou en tête de colonne de distillation.

Pour rendre les inhibiteurs plus efficaces il convient d'injecter en pied de colonne de l'oxygène, de l'air ou de l'air dit appauvri à 7% O2. De façon préférée la quantité d'oxygène injectée correspond à une teneur de 0,2% à 0,5% rapportée à la quantité de vapeur organique dans la colonne.

La colonne peut opérer sous vide, afin de minimiser l'exposition thermique des composés thermosensibles au sein de la colonne. Avantageusement, la colonne d'étêtage fonctionne sous un vide allant de 1000 Pa à 30000 Pa.

Le flux de pied alimente de préférence la colonne permettant d'obtenir l'ester purifié en pied de colonne entre le plateau théorique 6 à 9.

La colonne de distillation du pur comporte un équivalent de 2 et 15 plateaux théoriques de préférence 6 à 12 étages théoriques. Les internes utilisés pour la colonne peuvent être des plateaux à clapets ou des plateaux perforés à déversoir, des plateaux à courants croisés comme les Dual Flow les Ripple Trays, les Turbo Grid Shell, ou du garnissage ordonné comme du garnissage structuré tel le Mellapack 250X de Sulzer.

Le flux de tête de la colonne est constitué de l' acrylate de butyle de haute pureté ayant comme spécifications une pureté en ester supérieure à 99,5%, une teneur en éther dibutylique inférieure à 500 ppm, et enfin une teneur en eau inférieure à 400 ppm.

La colonne fonctionne avec un taux de reflux (débit de liquide condensé retourné à la colonne/ débit de pur) compris entre un ratio de 1/8 à 1/1 de préférence 1/4. Comme la colonne d'étêtage, celle-ci est stabilisée et de l'air ou de l'air appauvri (7%O2) est injecté en bas de colonne. La colonne peut opérer sous vide, afin de minimiser l'exposition thermique des composés thermosensibles au sein de la colonne. Avantageusement, la colonne de pur fonctionne sous un vide allant de 1000 Pascal à 20000 Pascal.

Avantageusement la température de fonctionnement est comprise entre 50°C et 160 °C.

Le flux de pied est concentré sur un évaporateur (non représenté) à film raclé afin de récupérer et renvoyer à l'alimentation de la colonne de rectification l'acrylate de butyle qui se trouvait en pied de cette colonne de rectification et d'alimenter le craqueur objet de l'invention avec le pied de celui-ci. Ce résidu alimente un réacteur à recirculation forcée comprenant un échangeur externe. La température du milieu réactionnelle est comprise entre 220° et 300°C de préférence 230°C à 280°C. La pression dans ce réacteur est maintenue entre 50k Pa et 300 kPa. Le produit de pied constitue le résidu ultime et il est envoyé vers la filière adéquate. Le produit de tête condensé à une température de 20°C à 30°C est envoyé à l'entrée de la colonne d'étêtage. Il n'est pas nécessaire d'injecter dans ce réacteur de l'air ou de l'air appauvri car le produit de pied provenant de l'évaporateur contient l'ensemble des stabilisants mis en œuvre dans le procédé.

Les exemples ci-après illustrent la présente invention sans toutefois en limiter la portée.

### PARTIE EXPERIMENTALE

Dans les exemples, les pourcentages sont indiqués en poids sauf indication contraire et les abréviations suivantes ont été utilisées :
AA : acide acrylique
ABU : acrylate de butyle
BuOH: butanol
BPB : butoxypropionate de butyle
DBE : éther dibutylique

### Exemple 1 Procédé de craquage catalytique suivant le brevet FR290172

On utilise un bouilleur thermosiphon en verre d'une capacité utile de 92 cm³.

Le bouilleur est alimenté en continu à partir d'un récipient de lourds ABU préalablement distillé à l'aide d'une pompe à membrane équipée d'un clapet de contrepression. Le flux d'alimentation est envoyé dans le bouilleur à température ambiante et la pression est maintenue à la pression atmosphérique. Le débit d'alimentation est régulé par mesure en continu de la masse de mélange initial. Le bouilleur est chauffé à l'aide de 3 colliers chauffants. Un doigt de gant de diamètre 10 mm mesure la température dans le milieu réactionnel. On ajuste la puissance de chauffe de manière à avoir la température voulue dans le bouilleur. Les vapeurs sortant du craqueur sont dirigées vers un condenseur refroidi à l'eau et le distillat est dirigé vers un bac de récupération à pression atmosphérique.

Pour une composition comprenant 70,5% de BPB, 10,7% d'ABU catalysée par 4,3% d'acide sulfurique, un temps de séjour de 30 min et une température de 171°C dans le craqueur, la teneur en éther dibutylique est de 8% dans le produit en tête du craqueur. Le réacteur est propre.

### Exemple 2 Procédé de craquage catalytique

On utilise un bouilleur à recirculation forcée d'un volume de 40 l alimenté en continu à l'aide d'une pompe à membrane par des lourds ABU placés sur une balance. Le débit d'alimentation est mesuré à l'aide d'un débitmètre massique placé sur la ligne d'alimentation et aussi par la variation de la masse indiquée par la balance au cours du temps. L'opération est réalisée à pression atmosphérique. La température du milieu réactionnel ainsi que celle à l'entrée et à la sortie de l'échangeur sont mesurées en continu. Le fluide caloporteur pour amener les calories à l'échangeur provient d'une chaudière à huile. La puissance de chauffe a été fixée de façon à avoir 65% de débit d'évaporation.

Dans les conditions opératoires suivantes : teneur massique en Acide Para Toluène Sulfonique dans l'alimentation : 1,5% ; P atmosphérique ; T de la chaudière à huile : 200°C, un temps de séjour de 10h exprimé comme le rapport de la boucle réactionnel/ débit d'alimentation, le taux de distillat est de 65% et ce dernier comprend 1,4% d'éther dibutylique. Par ailleurs, l'échantillon du produit de pied comprend des particules solides.

### Exemple 3 Procédé de craquage thermique selon l'invention

On utilise un bouilleur à recirculation forcée d'un volume de 40 L alimenté en continu à l'aide d'une pompe à membrane par des lourds ABU placés sur une balance. Le débit d'alimentation est mesuré à l'aide d'un débitmètre massique placé sur la ligne d'alimentation et aussi par la variation de la masse indiquée par la balance au cours du temps. L'opération est réalisée à une pression que l'on ajuste afin de ne pas vaporiser le butoxypropionate de butyle. La température du milieu réactionnel ainsi que celle à l'entrée et à la sortie de l'échangeur sont mesurées en continu. Le fluide caloporteur pour amener les calories à l'échangeur provient d'une chaudière à huile. La puissance de chauffe est fixée afin de garder la température d'essai fixée.

Les lourds ABU ont été préalablement distillés sous vide et contiennent environ 2000 ppm de Phénothiazine.

Le tableau 2 ci-dessous présente les résultats obtenus :

**[Tableau 2]**

| Essais | 1 | 2 | 3 | 4 |
|---|---|---|---|---|
| T réacteur (°C) | 230 | 235 | 240 | 245 |
| Temps (h) | 10 | 10 | 10 | 10 |
| Taux d'évaporation (%) | 41 | 43 | 47 | 50 |
| % DBE (ppm) | 2300 | 1356 | 1202 | 1050 |

Dans ces conditions opératoires la teneur en éther dibutylique est bien plus faible que ceux mentionnés dans les exemples 1 et 2. Le produit de pied est limpide.

## Revendications

1. Procédé de fabrication d'acrylate de butyle par estérification directe de l'acide acrylique par du butanol en présence d'acide sulfurique comme catalyseur, conduisant à l'obtention d'un mélange réactionnel brut contenant de l'acrylate de butyle, acide acrylique et butanol résiduels, sulfate acide de butyle, des traces d'acide sulfurique et des impuretés résultant des réactions secondaires, ledit procédé comprenant des étapes de neutralisation et de lavage à l'eau conduisant à l'obtention d'un mélange réactionnel exempt d'impuretés acides, **caractérisé en ce que** ledit mélange réactionnel lavé d'impuretés acides est soumis au moins aux étapes suivantes :
i) étêtage dans une colonne de distillation permettant d'obtenir :
- en tête un flux composé essentiellement des réactifs non réagis ;
- en pied un flux comprenant l'ester recherché et des sous-produits lourds ;
ii) on soumet le flux de pied de la colonne d'étêtage à une colonne de rectification permettant de séparer :
- en tête l' acrylate de butyle purifié ;
- en pied un flux contenant des sous-produits lourds, qui est concentré sur un évaporateur à film ou distillé dans une colonne d'équeutage afin de recycler à l'alimentation de la colonne de rectification -les composés légers présents, et d'éliminer un résidu final de sous-produits lourds,
iii) on soumet le flux de pied de la colonne de rectification à un traitement thermique effectué en l'absence de catalyseur dans un craqueur placé en sortie de l'évaporateur, permettant de séparer :
- en tête, un flux de produits valorisables recyclé à l'alimentation de la colonne d'étêtage ;
- en pied, un résidu envoyé vers une station de traitement.

2. Procédé selon la revendication 1, dans lequel lesdites impuretés acides sont l'acide sulfurique, le sulfate acide de butyle, le dimère d'acide acrylique et l'acide acrylique résiduel.

3. Procédé selon l'une quelconque des revendications 1 et 2, dans lequel le craquage est effectué à une température de 220°C à 300°C, et de préférence entre 230°C to 280°C.

4. Procédé selon l'une quelconque des revendications 1 à 3, dans lequel le craquage est effectué à une pression comprise entre 50000 Pa à 300000 Pa.

5. Procédé selon l'une quelconque des revendications 1 à 4, dans lequel le craquage est effectué en mode continu.

6. Procédé selon l'une quelconque des revendications 1 à 5, dans lequel le craqueur est un réacteur tubulaire, un réacteur agité avec une double enveloppe ou ayant une boucle de chauffage externe avec une circulation forcée.

7. Procédé selon l'une quelconque des revendications 1 à 6, dans lequel l'acrylate de butyle obtenu a une pureté supérieure à 99,5%, une teneur en éther dibutylique inférieure à 500 ppm, et une teneur en eau inférieure à 400 ppm.

## Patentansprüche

1. Verfahren zur Herstellung von Butylacrylat durch direkte Veresterung von Acrylsäure durch Butanol in Gegenwart von Schwefelsäure als Katalysator, was zum Erhalten eines rohen Reaktionsgemisches führt, das Butylacrylat, restliche Acrylsäure und restliches Butanol, Butylhydrogensulfat, Schwefelsäurespuren und aus Nebenreaktionen resultierende Verunreinigungen enthält, wobei das Verfahren Schritte zum Neutralisieren und Waschen mit Wasser umfasst, die zum Erhalten eines Reaktionsgemisches führen, das frei von sauren Verunreinigungen ist, **dadurch gekennzeichnet, dass** das von sauren Verunreinigungen gewaschene Reaktionsgemisch mindestens den folgenden Schritten unterzogen wird:
i) Abtrennen der Kopffraktion in einer Destillationskolonne, was es ermöglicht, Folgendes zu erhalten:
- am Kopf einen Strom, der im Wesentlichen aus nicht umgesetzten Edukten besteht;
- am Sumpf einen Strom, der den angestrebten Ester und schwerflüchtige Nebenprodukte umfasst;
ii) der Sumpfstrom der Kolonne zum Abtrennen der Kopffraktion wird einer Rektifikationskolonne unterzogen, was es ermöglicht, Folgendes abzutrennen:
- am Kopf das gereinigte Butylacrylat;
- am Sumpf einen schwerflüchtige Nebenprodukte enthaltenden Strom, der in einem Filmverdampfer konzentriert wird oder in einer Kolonne zum Abtrennen der Sumpffraktion destilliert wird, um die vorhandenen leichtflüchtigen Verbindungen zum Zulauf der Rektifikationskolonne zurückzuführen und einen endgültigen Rückstand von schwerflüchtigen Nebenprodukten zu eliminieren,
iii) der Sumpfstrom der Rektifikationskolonne wird einer thermischen Behandlung unterzogen, die ohne Katalysator in einem am Auslauf des Verdampfers angeordneten Cracker durchgeführt wird, was es ermöglicht, Folgendes abzutrennen:
- am Kopf einen Strom aus verwertbaren Produkten, der zum Zulauf der Kolonne zum Abtrennen der Kopffraktion zurückgeführt wird;
- am Sumpf einen Rückstand, der zu einer Behandlungsstation geführt wird.

2. Verfahren nach Anspruch 1, wobei die sauren Verunreinigungen Schwefelsäure, das Butylhydrogensulfat, das Acrylsäuredimer und die restliche Acrylsäure sind.

3. Verfahren nach einem der Ansprüche 1 und 2, wobei das Cracken bei einer Temperatur von 220 °C bis 300 °C und bevorzugt zwischen 230 °C und 280 °C durchgeführt wird.

4. Verfahren nach einem der Ansprüche 1 bis 3, wobei das Cracken bei einem Druck zwischen 50000 Pa und 300000 Pa durchgeführt wird.

5. Verfahren nach einem der Ansprüche 1 bis 4, wobei das Cracken im kontinuierlichen Betrieb durchgeführt wird.

6. Verfahren nach einem der Ansprüche 1 bis 5, wobei der Cracker ein Rohrreaktor, ein Rührreaktor mit einem doppelten Mantel oder mit einem externen Heizkreislauf mit Zwangsumlauf ist.

7. Verfahren nach einem der Ansprüche 1 bis 6, wobei das erhaltene Butylacrylat eine Reinheit über 99,5 %, einen Gehalt an Dibutylether unter 500 ppm und einen Wassergehalt unter 400 ppm hat.

## Claims

1. A process for producing butyl acrylate by direct esterification of acrylic acid with butanol in the presence of sulfuric acid as catalyst, resulting in production of a crude reaction mixture containing butyl acrylate, residual acrylic acid and residual butanol, butyl hydrogen sulfate, traces of sulfuric acid and impurities resulting from side reactions, said process comprising steps of neutralization and washing with water leading to the production of a reaction mixture free of acidic impurities, **characterized in that** said reaction mixture washed of acidic impurities is subjected at least to the following steps:
i) topping in a distillation column to obtain:
- at the top, a stream composed essentially of unreacted reagents;
- at the bottom, a stream comprising the desired ester and heavy by-products;
ii) the bottom stream from the topping column is subjected to a rectification column to separate:
- at the top, purified butyl acrylate;
- at the bottom, a stream containing heavy by-products, which is concentrated on a film evaporator or distilled in a tailing column in order to recycle the light compounds present to the rectification column feed, and to remove a final residue of heavy by-products;
iii) the bottom stream from the rectification column is subjected to a heat treatment carried out in the absence of catalyst in a cracker placed at the outlet of the evaporator, to separate:
- at the top, a stream of upgradable products recycled to the topping column feed;
- at the bottom, a residue sent to a treatment plant.

2. The process as claimed in claim 1, wherein said acidic impurities are sulfuric acid, butyl hydrogen sulfate, acrylic acid dimer and residual acrylic acid.

3. The process as claimed in either one of claims 1 and 2, wherein the cracking is carried out at a temperature of 220°C to 300°C, and preferably between 230°C to 280°C.

4. The process as claimed in any one of claims 1 to 3, wherein the cracking is carried out at a pressure of between 50 000 Pa to 300 000 Pa.

5. The process as claimed in any one of claims 1 to 4, wherein the cracking is carried out in a continuous mode.

6. The process as claimed in any one of claims 1 to 5, wherein the cracker is a tubular reactor, a jacketed stirred reactor or a reactor having an external heating loop with forced circulation.

7. The process as claimed in any one of claims 1 to 6, wherein the butyl acrylate obtained has a purity of greater than 99.5%, a dibutyl ether content of less than 500 ppm, and a water content of less than 400 ppm.
